# EUROPÄISCHE PATENTSCHRIFT

(11) **EP 3 271 118 B1**
(45) Veröffentlichungstag und Bekanntmachung des Hinweises auf die Patenterteilung: **27.02.2019**
(21) Anmeldenummer: 16712295.1
(22) Anmeldetag: 17.03.2016
(51) Int. Cl.: A61B 1/00, A61B 34/30, A61B 90/00, A61B 34/00, B25J 19/02, B25J 19/06, B25J 9/16

(54) **ROBOTERSYSTEM UND VERFAHREN ZUM BETRIEB EINES TELEOPERATIVEN PROZESSES**
ROBOT SYSTEM AND METHOD FOR OPERATING A TELEOPERATIVE PROCESS
SYSTÈME ROBOTIQUE ET PROCÉDÉ DE FONCTIONNEMENT D'UN PROCESSUS COMMANDÉ PAR TÉLÉOPÉRATEUR

(30) Priorität: 18.03.2015 DE 102015204867
(43) Veröffentlichungstag der Anmeldung: 24.01.2018
(73) Patentinhaber: KUKA Deutschland GmbH, 86165 Augsburg (DE)
(72) Erfinder: KOGAN, Yevgen, 86152 Augsburg (DE)
(74) Vertreter: Mader, Joachim
(86) Internationale Anmeldenummer: PCT/EP2016/055851
(87) Internationale Veröffentlichungsnummer: WO 2016/146768

(56) Entgegenhaltungen:
- EP-A1- 0 812 662
- WO-A2-2005/039836
- DE-A1-102011 009 814
- DE-A1-102014 108 956

## Beschreibung

### 1. Technischer Bereich

Die vorliegende Erfindung betrifft ein Robotersystem und ein Verfahren zum Betrieb eines teleoperativen Prozesses, wie etwa zur roboterunterstützten Bearbeitung von Werkstücken oder zur medizinischen Behandlung eines Patienten, bei dem ein robotergeführtes Werkzeug und ein Bildaufnahmegerät zum Einsatz kommen.

### 2. Technischer Hintergrund

Die Telerobotik ist ein Teilgebiet der Robotik, das sich mit der Steuerung von Robotern aus der Distanz beschäftigt. Ferngesteuerte Manipulatoren werden auch als Teleoperatoren bezeichnet und die Telerobotik kommt in vielen industriellen Anwendungen zum Einsatz. Ein weiteres Anwendungsgebiet ist die Medizin, wobei insbesondere in der Chirurgie roboterassistierte Systeme teleoperativ betrieben werden. Beispielsweise zählen mit Bildaufnahmegeräten, wie z.B. Ultraschallgeräten oder endoskopischen Bildgebungsgeräten, gestützte medizinische Untersuchungen bzw. Behandlungen heute zu den Standardeingriffen in der Medizin. Ein Beispiel für eine derartige medizinische Behandlung ist die minimal-invasive Chirurgie, bei der als Werkzeuge endoskopische Instrumente, z.B. Biopsienadeln, zum Einsatz kommen und wobei der Einsatz dieser medizinischen Instrumente im Körper eines Patienten mit Bildaufnahmegeräten, z.B. einem Videoendoskop, überwacht wird. Bei derartigen Eingriffen muss das medizinische Instrument im vom Bildaufnahmegerät erfassten Bereich bleiben, damit der Operateur bzw. Chirurg eine optische Rückkopplung seiner Arbeit hat. Bei solchen Systemen kann es jedoch passieren, dass durch ungünstige Positionierung des Bildaufnahmegeräts oder des Instruments, das Instrument während der Operation aus dem erfassten Bereich heraus bewegt wird, wie z.B. aus dem Kamerasichtfeld des Videoendoskops. In diesem Fall muss die Kamera bzw. das Instrument so positioniert werden, dass das Instrument wieder sichtbar wird, wobei es (von außen des Patientenkörpers) nicht immer offensichtlich ist, wie die Kamera bzw. das Instrument bewegt werden müssen. Eine solche Situation sollte also möglichst vermieden werden. Ähnliche Probleme existieren in einer Vielzahl von anderen teleoperativen Prozessen.

Aus dem Stand der Technik sind Verfahren bekannt, bei denen ein Bildaufnahmegerät, nämlich ein Ultraschallkopf, mittels eines Manipulators, insbesondere eines Roboters, geführt wird. Beispielsweise ist aus der US 7,753,851 ein Robotersystem vorbekannt, bei dem eine Sonde am Handflansch des Roboters angebracht ist, und vom Roboter bewegt werden kann. Im Vergleich zum manuellen Bedienen der Sonde erlaubt die roboterunterstützte Behandlung eine besonders präzise Orientierung der Sonde.

Aus der US 6,236,875 B1 ist vorbekannt eine vordefinierte Sicherheitszone im Körper eines Patienten (z.B. eine Umgebung um ein bestimmtes Organ des Patienten) vorzugeben und zu überwachen. Die Position des für den Eingriff verwendeten medizinischen Instruments wird ebenfalls überwacht und das Instrument wird deaktiviert, wenn es sich der Sicherheitszone nähert.

In der US 2013/0038707A1 ist ein Verfahren beschrieben, um einem Chirurgen mittels einer Videokamera die Möglichkeit des optischen Feedbacks während eines minimal-invasiven chirurgischen Eingriffs zu bieten. Dabei soll auch das verwendete medizinische Instrument überwacht werden, um ein Eintreten des Instruments in eine vordefinierte Sicherheitszone im Körper des Patienten zu verhindern.

Ein Nachteil von einigen der obigen Verfahren ist, dass die Definition von Sicherheitszonen eine aufwendige Planung voraussetzt, bei der insb. die jeweiligen Sicherheitszonen für jeden Fall individuell bestimmt werden müssen. Zudem muss der Körper des Patienten während der Operation entweder fixiert oder getrackt werden, damit seine aktuelle Position den vordefinierten Sicherheitszonen entspricht. Zudem ist es auch in diesen Fällen möglich, dass das medizinische Instrument den erfassten Bereich der Bildaufnahmegeräte verlässt, also z.B. das Sichtfeld der verwendeten Kamera, da das Bildaufnahmegerät als auch das Werkzeug meist beweglich sind. Diese Nachteile existieren analog auch in anderen, z.B. industriellen Anwendungen.

Es ist daher die Aufgabe der vorliegenden Erfindung ein verbessertes System und Verfahren zum Betrieb eines teleoperativen Prozesses bereitzustellen, wie etwa zur roboterunterstützten medizinischen Behandlung eines Patienten, mit dem die Nachteile des Standes der Technik vermieden oder vermindert werden können. Es ist insbesondere eine Aufgabe der vorliegenden Erfindung automatisch geeignete Gegenmaßnahmen bereitzustellen, wenn ein Werkzeug den erfassten Bereich eines Bildaufnahmegeräts zu verlassen droht.

Diese und weitere Aufgaben, welche aus der folgenden detaillierten Beschreibung deutlicher werden, werden durch den Gegenstand der unabhängigen Ansprüche 1 und 7 gelöst.

### 3. Inhalt der Erfindung

Die vorliegende Erfindung betrifft insbesondere ein Robotersystem für einen teleoperativen Prozess, bei dem ein Bildaufnahmegerät und ein Werkzeug vorgesehen sind, wobei das Werkzeug von einem ersten Manipulator geführt ist. Der Manipulator ist insbesondere ein mehrachsiger Gelenkarmroboter. Insbesondere können die Achsen des Manipulators mit Sensoren zur Erfassung der an den Achsen wirkenden Kräfte und/oder Drehmomente versehen sein. Das System umfasst dabei eine Steuereinrichtung, die eingerichtet ist, um den aktuell erfassten Bereich des Bildaufnahmegeräts zu bestimmen, und die Lage des Werkzeugs (wie z.B. die Spitze einer Nadel oder eines Schweißdrahts) relativ zum Bildaufnahmegerät zu bestimmen, und eine Aktion auszuführen, wenn das Werkzeug Grenzen des aktuell erfassten Bereichs des Bildaufnahmegeräts verletzt. Unter dem aktuell erfassten Bereich ist derjenige Bereich im Raum gemeint, den das Bildaufnahmegerät in seiner jeweiligen Position und Orientierung erfassen kann. Durch die Kenntnis der Lage des Werkzeugs relativ zum Bildaufnahmegerät, ist es der Steuereinrichtung möglich zu bestimmen, ob und wie das Werkzeug von dem Bildaufnahmegerät erfasst wird. Dabei ist es für viele Anwendungen ausreichend, wenn nur ein bestimmter Teil des Werkzeugs erfasst wird, wie etwa die Spitze eines Werkzeugs, die einen bestimmten Bereich nicht betreten oder verlassen soll, oder irgendein anderes Teil des Werkzeugs. Daher wird hierin der Schritt des "Bestimmens der Lage des Werkzeugs" breit verstanden und umfasst auch jegliche Bestimmung der Lage nur eines Teils des Werkzeugs. Grundsätzlich ist es vorteilhaft möglich die Grenzen des aktuell erfassten Bereichs je nach Anwendung zu definieren. Beispielsweise lässt am Rand des erfassten Bereichs die Qualität der Visualisierung in der Regel nach. Abhängig von der Anwendung, d.h. der benötigten Qualität der Visualisierung, kann daher die Grenze ab der bspw. ein Alarm ausgegeben wird, eingestellt werden.

Die Sensoren zur Erfassung der an den Achsen wirkenden Kräfte und/oder Drehmomente können beispielsweise Strom- und/oder Spannungssensoren sein, welche den Strom/die Spannung der Antriebe der Achsen des Manipulators überwachen, um die an den Achsen wirkenden Kräfte und/oder Drehmomente zu erfassen. Ebenso können die Sensoren resistive, kapazitive und/oder induktive Kraft-und/oder Drehmomenten-Sensoren sein. Weiterhin können Piezosensoren oder andere bekannte Sensoren verwendet werden, welche die Erfassung von Kräften und/oder Drehmomenten ermöglichen.

Vorzugweise umfasst die Aktion das Ausgeben eines Warnhinweises und/oder eine Stillsetzung bzw. Zähschaltung des ersten Manipulators und/oder ein Deaktivieren des Werkzeugs. Diese Aktionen können je nach Situation auch gestaffelt erfolgen. So ist es beispielsweise vorteilhaft, wenn ein Alarmsignal, z.B. optisch oder akustisch, in einer ersten Stufe ausgebeben wird, wenn das Werkzeug dem Rand des erfassten Bereichs zu nahe kommt. Stellt die Steuerung fest, dass das Werkzeug nach der Warnung immer noch näher an den Rand des erfassten Bereichs geführt wird, wird z.B. in einer zweiten Stufe das Werkzeug, wie z.B. ein Elektrotomie-Gerät oder eine Schweiß-Elektrode, deaktiviert. Erst als letzte Sicherheitsmaßnahme wird der erste Manipulator stillgesetzt. Dabei wird hierin das Stillsetzen als eine Unterform des Zähschaltens verstanden. Beim Zähschalten werden die Bewegungen des Manipulators künstlich erschwert, so dass ein Anwender z.B. ein Chirurg über einen Force-Feedback-Controller, mit dem er den Manipulator steuert, eine haptische Rückkopplung erhält. Das Zähschalten kann die Bewegung des Manipulators derart erschweren, dass der Manipulator praktisch stillgesetzt ist. Eine Stillsetzung kann natürlich auch durch eine Aktivierung der mechanischen Bremsen des Manipulators erfolgen, oder durch andere geeignete Maßnahmen.

Weiter vorzugsweise ist das Bildaufnahmegerät von einem zweiten Manipulator, insbesondere einem mehrachsigen Gelenkarmroboter, geführt. Die ersten und zweiten Manipulatoren kooperieren und die Steuereinrichtung ist vorteilhaft in der Lage, Positionsinformationen beider Manipulatoren abzufragen. Ein mehrachsiger Gelenkarmroboter ist bevorzugt, da dieser einen hohen Freiheitsgrad bietet und das Bildaufnahmegerät bzw. das Werkzeug sehr flexibel führen kann.

Bevorzugt ist die Steuereinrichtung weiter eingerichtet, um den aktuell erfassten Bereich des Bildaufnahmegeräts mittels der Manipulatorsteuerung des zweiten Manipulators zu bestimmen. Die Steuereinrichtung ist beispielsweise eingerichtet, um Positionsinformationen des zweiten, und vorzugsweise auch des ersten, Manipulators abzufragen bzw. zu verarbeiten. Mit diesen Positionsinformationen ist es möglich die Lage und Orientierung des Bildaufnahmegeräts im Raum zu berechnen. Da der physikalisch erfassbare, bzw. Sichtbereich des Bildaufnahmegeräts bekannt ist, lässt sich somit der aktuell erfasste Bereich des Bildaufnahmegeräts relativ einfach bestimmen.

Besonders bevorzugt ist die Steuereinrichtung weiter eingerichtet, um die Lage des Werkzeugs mittels der Manipulatorsteuerung des ersten Manipulators zu bestimmen. Die Manipulatorsteuerung stellt z.B. die Positionsinformationen des ersten Manipulators zur Verfügung. Da die Anordnung bzw. Befestigung des Werkzeugs am Manipulator vorgegeben ist, kann somit die exakte Lage des Werkzeugs im Raum einfach ermittelt werden. Besonders vorteilhaft ist es, wenn zwei Manipulatoren verwendet werden und die Steuereinrichtung auf die Positionsinformationen beider Manipulatoren zugreifen kann. Wenn die relative Position der beiden Manipulatoren zueinander bekannt ist, was einfach ermittelt werden kann, ist es der Steuereinrichtung somit möglich, direkt die relative Lage des Werkzeugs zum Bildaufnahmegerät, und damit letztendlich zum aktuell erfassten Bereich des Bildaufnahmegeräts, zu bestimmen.

Vorzugsweise sind die verwendeten Manipulatoren, also der erste Manipulator und der zweite, wenn das Bildaufnahmegerät von einem zweiten Manipulator geführt ist, mehrachsige Gelenkarmroboter, deren Achsen vorzugsweise mit Sensoren zur Erfassung der an den Achsen wirkenden Kräfte und/oder Drehmomente versehen sind. Mit Hilfe der Sensoren lassen sich für jeden Manipulator Kraft-Grenzen definieren, die er nicht überschreiten darf, wenn er bspw. das Bildaufnahmegerät gegen den Körper eines Patienten drückt.

Vorzugweise ist das Bildaufnahmegerät eine Ultraschallsonde oder ein endoskopisches Bildgebungsgerät. Im Falle einer Ultraschallsonde wird diese in der Regel am Körper des Patienten entlanggeführt, wofür sich besonders die oben erwähnten Gelenkarmroboter mit Sensoren eignen, da die Ultraschallsonden nur dann optimal arbeiten, wenn sie mit der richtigen Anpresskraft gegen den Körper des Patienten geführt werden.

Grundsätzlich bevorzugt ist die Steuereinrichtung eine eigenständige Computervorrichtung, welche mit der Manipulatorsteuerung des ersten und gegebenenfalls des zweiten Manipulators verbunden ist. Die Aufgaben der Steuereinrichtung können jedoch auch von z.B. der Manipulatorsteuerung des ersten oder gegebenenfalls des zweiten Manipulators erfüllt werden, sofern diese eine ausreichende Rechenkapazität aufweisen.

Die vorliegende Erfindung betrifft auch ein Verfahren zum Betrieb eines teleoperativen Prozesses, wie z.B. der roboterunterstützten medizinischen Behandlung eines Patienten, bei dem in einem Schritt die Lage eines Werkzeugs (wie etwa eines medizinischen Instruments) bestimmt wird, welches von einem ersten Manipulator, insbesondere einem mehrachsigen Gelenkarmroboter, geführt wird. Die Lagebestimmung ist wie oben ausgeführt dann sehr einfach möglich, da die aktuellen Positionsdaten des ersten Manipulators aus der Manipulatorsteuerung bekannt sind. Weiter wird bei dem Verfahren der aktuell erfasste Bereich eines Bildaufnahmegeräts, wie etwa einer Ultraschallsonde oder eines Videoskops, bestimmt, welches Bildaufnahmegerät dazu dient, einem Anwender (z.B. einem Chirurgen) eine Visualisierung der Lage des Werkzeugs zu bieten, wie z.B. die Visualisierung der Lage der Spitze des Werkzeugs. Wenn das Bildaufnahmegerät ebenfalls von einem (zweiten) Manipulator geführt wird, ist diese Bestimmung einfach anhand der Positionsdaten aus der Manipulatorsteuerung möglich. Es ist ebenfalls möglich, das Bildaufnahmegerät mit z.B. Markern zu versehen. Die Marker am Bildaufnahmegerät werden dann bspw. von einem geeigneten Sensor erfasst, um die Lage des Markers im Raum, und damit - da der Offset von Marker und Bildaufnahmegerät bekannt ist - die Lage des Bildaufnahmegerät erfassen zu können.

In einem weiteren Schritt wird die Lage des Werkzeugs, wie etwa die Lage der Spitze einer Schweiß-Elektrode, relativ zum Bildaufnahmegerät bestimmt, und ein Warnhinweis ausgegeben und/oder der erste Manipulator stillgesetzt bzw. zähgeschaltet und/oder das Werkzeug deaktiviert, wenn das Werkzeug Grenzen des aktuell erfassten Bereichs des Bildaufnahmegeräts verletzt. Wie oben erläutert, können diese Grenzen anwendungsfallspezifisch festgelegt werden. Bei hohen Anforderungen an die Qualität der Visualisierung, kann z.B. bereits frühzeitig ein Warnsignal an den Anwender ausgegeben werden, bevor das Werkzeug den Rand des physikalisch erfassbaren Bereichs des Bildaufnahmegeräts erreicht hat, in dem zwar noch ein Bild erzeugt werden kann, dessen Qualität jedoch evtl. nicht mehr ausreichend ist.

Grundsätzlich bevorzugt wird auch bei dem Verfahren das Bildaufnahmegerät von einem zweiten Manipulator, insbesondere einem mehrachsigen Gelenkarmroboter, geführt. Dies erlaubt vorteilhaft, dass der aktuell erfasste Bereich des Bildaufnahmegeräts mittels der Manipulatorsteuerung des zweiten Manipulators bestimmt werden kann. Die Manipulatorsteuerung verfügt über genaue und aktuelle Positionsdaten des zweiten Manipulators und da die relative Lage des Bildaufnahmegeräts zum zweiten Manipulator, bzw. zum Koordinatensystem des Manipulators, bekannt ist, kann somit einfach der erfasste Bereich des Bildaufnahmegeräts berechnet werden.

Vorzugsweise wird außerdem die Lage des Werkzeugs mittels der Manipulatorsteuerung des ersten Manipulators bestimmt.

Aus den oben erwähnten Gründen ist es weiter bevorzugt, wenn bei dem erfindungsgemäßen Verfahren mehrachsige Gelenkarmroboter zum Einsatz kommen, wobei die Achsen der Gelenkarmroboter mit Sensoren zur Erfassung der an den Achsen wirkenden Kräfte und/oder Drehmomente versehen sind.

### 4. Ausführungsbeispiel

Im Folgenden wird die vorliegende Erfindung anhand eines nicht beschränkenden Ausführungsbeispiels beschrieben. Es zeigt:

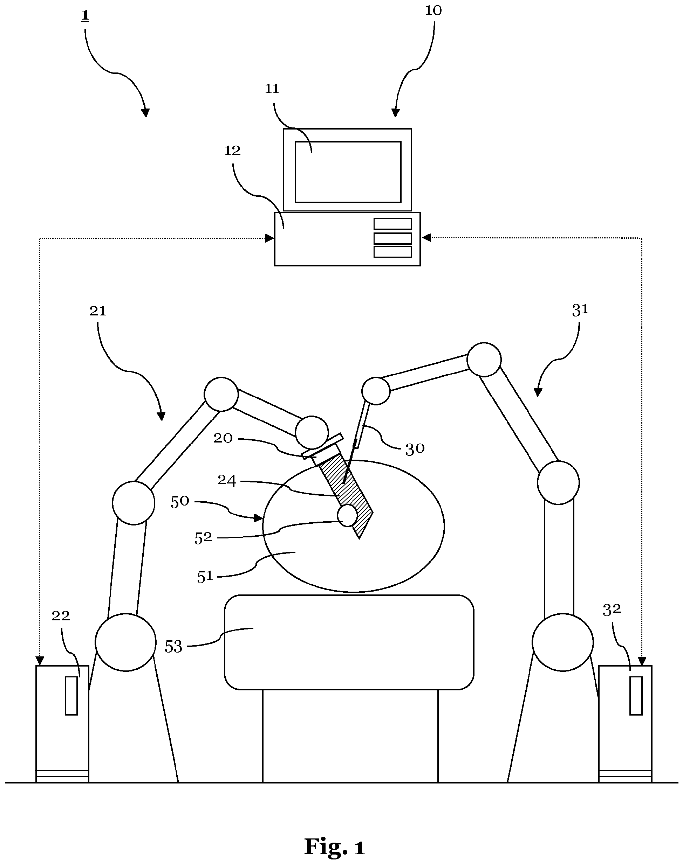

Fig. 1 schematisch ein erfindungsgemäßes System zur roboterunterstützten Behandlung eines Patienten.

In Figur 1 ist schematisch und beispielhaft ein erfindungsgemäßes System 1 zum roboterunterstützten Behandeln eines Patienten 50 illustriert. Dem Fachmann ist klar, dass die hierin beschriebenen Prinzipien natürlich auf jede Art der Teleoperation angewendet werden können, insbesondere auf Teleoperationen im industriellen Umfeld. Das System umfasst eine Steuereinrichtung 10, die einen Computer 12 und einen Bildschirm 11 aufweist. Der Patient 50 liegt auf einem Operationstisch 53 und in der gezeigten Darstellung soll Bezugszeichen 51 eine Schnittdarstellung durch den Hals des Patienten 50 andeuten. Im Hals 51 befindet sich ein zu untersuchender bzw. zu behandelnder Zielpunkt 52, wie etwa ein Tumor o.ä. Als Werkzeug dient in der Behandlung ein chirurgisches Instrument, nämlich eine Biopsienadel 30. Die Biopsienadel 30 wird von einem ersten Manipulator 31 geführt, der im gezeigten Fall ein mehrachsiger Gelenkarmroboter 31 ist. Dem Gelenkarmroboter 31 ist eine Manipulatorsteuerung 32 zugeordnet, die in Verbindung mit dem Computer 12 der Steuereinrichtung 10 ist, wie es durch die gestrichelten Pfeile angedeutet ist. Die Biopsienadel 30 soll zum Zielpunkt 52 geführt werden. Um dem Chirurgen die Führung der Biopsienadel 30 zu erleichtern, bzw. überhaupt zu ermöglichen, kommt ein Bildaufnahmegerät in Form einer Ultraschallsonde 20 zum Einsatz. Die Ultraschallsonde 20 wird von einem zweiten Manipulator 21 geführt, der ebenfalls ein mehrachsiger Gelenkarmroboter ist, und dem eine Manipulatorsteuerung 22 zugeordnet ist. Die Manipulatorsteuerung 22 und auch die Ultraschallsonde 20 sind mit der Steuereinrichtung 10 verbunden, wie es durch die gestrichelten Pfeile angedeutet wird.

Der Gelenkarmroboter 21 trägt und bewegt die Ultraschallsonde 20. Die Ultraschallsonde 20 wird vom Gelenkarmroboter 21 an den Körper des Patienten 50 gepresst, um Ultraschallbilder des Inneren des Körpers des Patienten zu machen. Die Ultraschallbilder werden an die Steuereinrichtung 10 bzw. den zugehörigen Computer 12 übertragen, im Computer 12 verarbeitet und dann am Bildschirm 11 angezeigt. Mit dem Bezugszeichen 24 soll der aktuell erfasste Bereich der Ultraschallsonde 20 (d.h. die Bildebene (Schallebene) der Ultraschallsonde) angezeigt werden. Die Bild- oder Schallebene der Sonde ist üblicherweise nur wenige Millimeter dick, so dass die Sonde sehr genau ausgerichtet werden muss, um aussagekräftige Bilder zu liefern. Das Ausrichten der Sonde und das Anpressen der Sonde erfolgt durch den Manipulator bzw. Gelenkarmroboter 21, so dass ein Chirurg von diesen Aufgaben entlastet ist. Zu diesem Zweck ist es vorteilhaft, wenn der Gelenkarmroboter 21 mit Kraftsensoren versehen ist und in Kraftregelung arbeitet, so dass er die Ultraschallsonde 20 mit einer definierten Kraft auf die Hautoberfläche des Patienten 50 presst.

Da die Lage der Ultraschallsonde 20 aufgrund der aktuellen Manipulatorposition feststeht bzw. daraus berechnet werden kann, und der Verlauf und die Orientierung des erfassten Bereichs 24 ebenfalls bekannt ist, ist es somit möglich, genau zu berechnen, wo sich der erfasste Bereich 24 im Raum befindet.

In Figur 1 ist die Spitze der Biopsienadel 30 innerhalb des aktuell erfassten Bereichs 24, so dass der Chirurg auf dem Bildschirm 11 die Bewegung der Spitze durch den Körper des Patienten 50 verfolgen und die Biopsienadel 20 entsprechend zielgerichtet zum Zielpunkt 52 führen kann. Die Lage und Orientierung der Biopsienadel ist aufgrund der Roboterposition und Pose des Manipulators 31 genau bekannt bzw. kann genau bestimmt werden. Da die Steuereinrichtung 10 aus den beiden Manipulatorsteuerungen 22 und 32 die jeweilige Position und Pose der beiden Manipulatoren 21 und 31 kennt, bzw. diese mit Hilfe (d.h. mittels) der Manipulatorsteuerungen berechnen kann, kann die Steuereinrichtung 10 die Lage der Biopsienadel 30 relativ zur Ultraschallsonde 20 bestimmen, und somit auch die relative Lage von z.B. der Spitze der Biopsienadel 30 zum aktuell erfassten Bereich 24. Dies erlaubt es der Steuereinrichtung festzustellen, wenn die Biopsienadel 30 Grenzen des erfassten Bereichs 24 der Ultraschallsonde 20 verletzt. Wenn eine derartige Grenzverletzung festgestellt wird, kann bspw. auf dem Bildschirm 11 eine entsprechende Warnung ausgegeben werden, oder der Manipulator 31 wird zähgeschaltet.

### Referenzzeichenliste:

- 1: System
- 10: Steuereinrichtung
- 11: Bildschirm
- 12: Computer
- 20: Bildaufnahmegerät (Ultraschallsonde)
- 21, 31: Manipulatoren (Gelenkarmroboter)
- 22, 32: Manipulatorsteuerungen
- 24: aktuell erfasster Bereich (Schallebene)
- 30: Werkzeug (Biopsienadel)
- 50: Patient
- 51: Querschnitt durch Hals
- 52: Zielpunkt
- 53: Operationstisch

## Patentansprüche

1. Ein Robotersystem (1) für einen teleoperativen Prozess, umfassend:
- ein Bildaufnahmegerät (20), und
- ein Werkzeug (30), wobei das Werkzeug (30) von einem ersten Manipulator (31), in Form von einem mehrachsigen Gelenkarmroboter, geführt ist, wobei die Achsen des Manipulators mit Sensoren zur Erfassung der an den Achsen wirkenden Kräfte und/oder Drehmomente versehen sind; und wobei das Robotersystem (1) weiter umfasst:
- eine Steuereinrichtung (10), die eingerichtet ist, um:
(a) den aktuell erfassten Bereich (24) des Bildaufnahmegeräts (20) zu bestimmen, und
(b) die Lage des Werkzeugs (30) relativ zum Bildaufnahmegerät (20) zu bestimmen, und
(c) eine Aktion auszuführen, wenn das Werkzeug (30) Grenzen des aktuell erfassten Bereichs (24) des Bildaufnahmegeräts (20) verletzt.

2. Das System (1) nach Anspruch 1, wobei die Aktion das Ausgeben eines Warnhinweises umfasst und/oder eine Stillsetzung bzw. Zähschaltung des ersten Manipulators (31) und/oder ein Deaktivieren des Werkzeugs.

3. Das System (1) nach Anspruch 1 oder 2, wobei das Bildaufnahmegerät (20) von einem zweiten Manipulator (21), insbesondere einem mehrachsigen Gelenkarmroboter, geführt ist.

4. Das System (1) nach dem vorhergehenden Anspruch, wobei die Steuereinrichtung (10) weiter eingerichtet ist, um den aktuell erfassten Bereich (24) des Bildaufnahmegeräts (20) mittels der Manipulatorsteuerung (22) des zweiten Manipulators (21) zu bestimmen.

5. Das System (1) nach einem der vorhergehenden Ansprüche, wobei die Steuereinrichtung (10) weiter eingerichtet ist, um die Lage des Werkzeugs (30) mittels der Manipulatorsteuerung (32) des ersten Manipulators (31) zu bestimmen.

6. Das System (1) nach einem der vorhergehenden Ansprüche, wobei das Bildaufnahmegerät (20) eine Ultraschallsonde oder ein endoskopisches Bildgebungsgerät ist.

7. Verfahren zum Betrieb eines teleoperativen Prozesses, umfassend die folgenden Schritte:
- Bestimmen der Lage eines Werkzeugs (30), welches Werkzeug (30) von einem ersten Manipulator (31), in Form von einem mehrachsigen Gelenkarmroboter, geführt wird, wobei die Achsen des Manipulators mit Sensoren zur Erfassung der an den Achsen wirkenden Kräfte und/oder Drehmomente versehen sind;
- Bestimmen des aktuell erfassten Bereichs (24) eines Bildaufnahmegeräts (20), welches Bildaufnahmegerät (20) dazu dient, einem Anwender eine Visualisierung der Lage des Werkzeugs (30) zu bieten; und
- Bestimmen der Lage des Werkzeugs (30) relativ zum Bildaufnahmegerät (20), und Ausgeben eines Warnhinweises und/oder Stillsetzung bzw. Zähschaltung des ersten Manipulators und/oder Deaktivieren des Werkzeugs, wenn das Werkzeug Grenzen des aktuell erfassten Bereichs (24) des Bildaufnahmegeräts (20) verletzt.

8. Das Verfahren nach Anspruch 7, wobei das Bildaufnahmegerät (20) von einem zweiten Manipulator (21), insbesondere einem mehrachsigen Gelenkarmroboter, geführt wird.

9. Das Verfahren nach dem vorhergehenden Anspruch, wobei der aktuell erfasste Bereich (24) des Bildaufnahmegeräts (20) mittels der Manipulatorsteuerung (22) des zweiten Manipulators (21) bestimmt wird.

10. Das Verfahren nach einem der vorhergehenden Verfahrensansprüche, wobei die Lage des Werkzeugs (30) mittels der Manipulatorsteuerung (32) des ersten Manipulators (31) bestimmt wird.

11. Das Verfahren nach einem der vorhergehenden Verfahrensansprüche, wobei das Bildaufnahmegerät (20) eine Ultraschallsonde oder ein endoskopisches Bildgebungsgerät ist.

12. Das System (1) oder Verfahren nach einem der vorhergehenden Ansprüche, wobei der Schritt des Bestimmens der Lage des Werkzeugs (30) relativ zum Bildaufnahmegerät (20) nur ein Bestimmen der Lage eines Teils des Werkzeugs umfasst.

## Claims

1. A robot system (1) for a teleoperative process, comprising:
- an image recording device (20), and
- a tool (30), wherein the tool (30) is guided by a first manipulator (31), in the form of a multi-axis articulated robot arm, wherein the axes of the manipulator are provided with sensors for detecting the forces and/or torques acting on the axes;
and wherein the robot system (1) further comprises:
- a control unit (10) that is configured to:
(a) determine the currently captured region (24) of the image recording device (20), and
(b) determine the pose of the tool (30) relative to the image recording device (20), and
(c) perform an action, when the tool (30) violates the limits of the currently captured region (24) of the image recording device (20).

2. The system (1), as claimed in claim 1, wherein the action comprises the output of a warning and/or a shutdown or, more specifically, hard switching of the first manipulator (31) and/or a deactivation of the tool.

3. The system (1), as claimed in claim 1 or 2, wherein the image recording device (20) is guided by a second manipulator (21), in particular, a multi-axis articulated robot arm.

4. The system (1), as claimed in the preceding claim, wherein the control unit (10) is further configured to determine the currently captured region (24) of the image recording device (20) by means of the manipulator controller (22) of the second manipulator (21).

5. The system (1), as claimed in any one of the preceding claims, wherein the control unit (10) is further configured to determine the pose of the tool (30) by means of the manipulator controller (32) of the first manipulator (31).

6. The system (1), as claimed in any one of the preceding claims, wherein the image recording device (20) is an ultrasound probe or an endoscopic imaging device.

7. Method for operating a teleoperative process, comprising the following steps of:
- determining the pose of a tool (30), which tool (30) is guided by a first manipulator (31), in the form of a multi-axis articulated robot arm, wherein the axes of the manipulator are provided with sensors for detecting the forces and/or torques acting on the axes;
- determining the currently captured region (24) of an image recording device (20), which image recording device (20) is used to offer a user a visualization of the pose of the tool (30); and
- determining the pose of the tool (30) relative to the image recording device (20), and emitting a warning and/or shutting down or, more specifically, hard switching the first manipulator and/or deactivating the tool, when the tool violates the limits of the currently captured region (24) of the image recording device (20).

8. The method, as claimed in claim 7, wherein the image recording device (20) is guided by a second manipulator (21), in particular, a multi-axis articulated robot arm.

9. The method, as claimed in the preceding claim, wherein the currently captured region (24) of the image recording device (20) is determined by means of the manipulator controller (22) of the second manipulator (21).

10. The method, as claimed in any one of the preceding method claims, wherein the pose of the tool (30) is determined by means of the manipulator controller (32) of the first manipulator (31).

11. The method, as claimed in any one of the preceding method claims, wherein the image recording device (20) is an ultrasound probe or an endoscopic imaging device.

12. The system (1) or method, as claimed in any one of the preceding claims, wherein the step of determining the pose of the tool (30) relative to the image capturing device (20) comprises only a determining of the pose of a part of the tool.

## Revendications

1. Un système de robot (1) pour un processus téléopératoire, comprenant :
- un appareil de prise de vues (20) et
- un outil (30), l'outil (30) étant guidé par un premier manipulateur (31) en forme de robot à bras articulé multiaxes, les axes du manipulateur étant munis de capteurs pour détecter les forces et/ou les couples de rotation agissant sur les axes ;
et le système de robot (1) comprenant en outre :
- un dispositif de commande (10) qui est agencé pour :
(a) déterminer la zone détectée instantanée (24) de l'appareil de prise de vues (20), et
(b) déterminer la position de l'outil (30) par rapport à l'appareil de prise de vues (20), et
(c) effectuer une action lorsque l'outil (30) dépasse des limites de la zone détectée instantanée (24) de l'appareil de prise de vues (20).

2. Le système (1) selon la revendication 1, dans lequel l'action comprend l'émission d'un signal d'alarme et/ou une mise à l'arrêt, respectivement un ralentissement, du premier manipulateur (31) et/ou une désactivation de l'outil.

3. Le système (1) selon la revendication 1 ou 2, dans lequel l'appareil de prise de vues (20) est guidé par un second manipulateur (21), en particulier par un robot à bras articulé multiaxes.

4. Le système (1) selon la revendication précédente, dans lequel le dispositif de commande (10) est agencé en outre pour déterminer la zone détectée instantanée (24) de l'appareil de prise de vues (20) au moyen de la commande de manipulateur (22) du second manipulateur (21).

5. Le système (1) selon l'une des revendications précédentes, dans lequel le dispositif de commande (10) est agencé en outre pour déterminer la position de l'outil (30) au moyen de la commande de manipulateur (32) du premier manipulateur (31).

6. Le système (1) selon l'une des revendications précédentes, dans lequel l'appareil de prise de vues (20) est une sonde à ultrasons ou un appareil d'imagerie endoscopique.

7. Procédé de mise en oeuvre d'un processus téléopératoire, comprenant les étapes suivantes :
- détermination de la position d'un outil (30), lequel outil (30) est guidé par un premier manipulateur (31) en forme de robot à bras articulé multiaxes, les axes du manipulateur étant munis de capteurs pour détecter les forces et/ou les couples de rotation agissant sur les axes ;
- détermination de la zone détectée instantanée (24) de l'appareil de prise de vues (20), lequel appareil de prise de vues (20) sert à offrir à un utilisateur une visualisation de la position de l'outil (30) ; et
- détermination de la position de l'outil (30) par rapport à l'appareil de prise de vues (20), et émission d'un signal d'alarme et/ou mise à l'arrêt, respectivement ralentissement, du premier manipulateur et/ou désactivation de l'outil lorsque l'outil dépasse des limites de la zone détectée instantanée (24) de l'appareil de prise de vues (20) .

8. Le procédé selon la revendication 7, dans lequel l'appareil de prise de vues (20) est guidé par un second manipulateur (21), en particulier par un robot à bras articulé multiaxes.

9. Le procédé selon la revendication précédente, dans lequel la zone détectée instantanée (24) de l'appareil de prise de vues (20) est déterminée au moyen de la commande de manipulateur (22) du second manipulateur (21) .

10. Le procédé selon l'une des revendications de procédé précédentes, dans lequel la position de l'outil (30) est déterminée au moyen de la commande de manipulateur (32) du premier manipulateur (31).

11. Le procédé selon l'une des revendications de procédé précédentes, dans lequel l'appareil de prise de vues (20) est une sonde à ultrasons ou un appareil d'imagerie endoscopique.

12. Le système (1) ou procédé selon l'une des revendications précédentes, dans lequel l'étape de détermination de la position de l'outil (30 par rapport à l'appareil de prise de vues (20) ne comprenant qu'une détermination de la position d'une partie de l'outil.
